# EUROPEAN PATENT SPECIFICATION

(11) **EP 1 521 562 B1**
(45) Date of publication and mention of the grant of the patent: **11.01.2012**
(21) Application number: 03724638.6
(22) Date of filing: 30.05.2003
(51) Int. Cl.: A61F 13/02, A61F 5/56, A63B 23/18, A63B 71/00, A61M 29/00

(54) **NASAL PASSAGE SPLINTS IMPROVEMENTS**
VERBESSERUNGEN VON NASENGANG-SPLINTS
AMELIORATIONS APPORTEES A DES ECARTEURS POUR VOIES NASALES

(30) Priority: 31.05.2002 AU PS275402
(43) Date of publication of application: 13.04.2005
(73) Proprietor: CNS, INC., Eden Prairie, MN 55344 (US)
(72) Inventor: STRATTON, Barrie, Bridgewater, S.A. 5155 (AU)
(74) Representative: Walker, Ralph Francis
(86) International application number: PCT/AU2003/000657
(87) International publication number: WO 2003/101359

(56) References cited:
- WO-A1-01/60294
- WO-A1-98/12998
- GB-A- 2 274 399
- US-A- 5 983 898

## Description

### FIELD OF INVENTION

This invention relates to a device to be applied to the nose to dilate the nose to assist breathing and more particularly to an applicator for such a device.

### BACKGROUND OF THE INVENTION

A nasal splint applicator as defined in the preamble of claim 1 is disclosed in WO-A-01/60294.

Nasal passage splints are described in my earlier US Patent Application Serial No. 09/929015 where there is disclosed a splint which adheres to one side only on the outside of a nose of a user and achieves retention of an open position of a passageway of the nose by having one part of the splint adhering to a portion of the nose overlying a relatively rigid structure and another part engaging a fleshy part of the nose and drawing it out by a lever action. In practice two splints would be used, one to each side of the nose, but they act independently.

One of the problems associated with such a splint is that they must be positioned relatively accurately on a nose by a user to be most effective.

As the splint is attached to the nose by adhesive it is preferable that the splint be able to be handled in such a manner that the fingers of a person applying the splint do not touch the adhesive surface.

An object of this invention is therefore to provide a splint and applicator assembly that can assist in the placement of the splints and thereby make the use of the splints more effective or to at least provide the public with a useful alternative.

### BRIEF DESCRIPTION OF THE INVENTION

In one form the invention is said to reside in a nasal splint applicator including:
a handle;
a pair of arms extending from the handle; and
a nasal splint removably attached to each of the arms;
wherein the handle and arms form a substantially Y shape, the handle being the base of the Y shape and the arms forming the wings of the Y shape and each nasal splint being adapted to be removably fastened to a respective side on the outside of the nose.

Preferably the nasal splints are integrally moulded with their respective arms with a line of weakness between them so that the nasal splint can be removed from the arm by breaking at the line of weakness.

Preferably the handle includes first and second grip portions joined by an integral hinge with respective arms extending from respective grip portions, whereby the handle can be moulded in a substantially elongate configuration and bent at the integral hinge to form the Y shaped position of the handle and arms.

There may be inter-engagable portions on each of the first and second grip portions which are adapted to engage when the grips are brought together into the handle and arm Y shaped position.

Preferably each nasal splint comprises an elongate resiliently deformable strut having inner and outer sides; a bend in the strut intermediate the ends to form an included angle in the strut on the outer side and first and second portions extending from the bend, an adhesive material on the inner side of the strut, the adhesive material being adapted to be removably secured to the external skin of the nose, wherein the splint is adapted for affixing longitudinally to only one side of the nose with the first portion removably secured to a rigid fleshy portion and the second portion removably secured to a soft fleshy portion and when so affixed causes a dilation of the nasal passage as a result of a lever effect exerted on the soft fleshy portion by the second portion of the strut.

Preferably each of the first and second portions of the strut are substantially planar.

Preferably the included angle on the outer side is in the range of from approximately 120 degrees to approximately 150 degrees.

Preferably the second portion of the strut of the nasal splint includes side wings and the adhesive material is also provided on the side wings.

In an alternative embodiment the first portion of the strut of the nasal splint can include the a further bend on the inner side of the strut. The bend can be a transverse bend in the strut.

Preferably the handle is joined to the nasal splint at a position on a side of the strut along the second portion.

In preference the handle is moulded integrally with the material of the splint from a plastics material.

In preference the connection between the handle and a splint is defined by a reduced thickness portion so that, subsequent to adhering of the splint to the side of a nose, the handle can be separated from its attachment to the splint.

In preference, the attachment is by reason of the shape of the plastic adjacent a side of the splint such that this creates a line of weakness so that by levering the handle with respect to the splint, the handle will break away.

In preference, there are two splints secured by a handle arrangement which has a middle portion and two sides.

The middle portion can be moulded from plastics material and have a shape including an integral hinge and two grips which are adapted to be brought together to provide and facilitate manually holding of the handle assembly and thereby inherently the splints.

Preferably the first portion of the strut of the nasal splint can include a concave bend on the inner side of the strut. The concave bend can be defined by a transverse bend in the strut.

Preferably the nasal splint includes a handle joined to the splint by a line of weakness such that after adherence of the splint to a side of the nose the handle can be separated from the splint by breaking along the line of weakness.

Preferably the handle and splint are integrally moulded.

### BRIEF DESCRIPTION OF THE DRAWINGS

For a better understanding of this invention it will now be described with the assistance of drawings wherein:
Figure 1 is a perspective view of a nasal splint and applicator assembly in an as moulded position;
Figure 2 is a plan view of the arrangement as in Figure 1
Figure 3 is a side view of the arrangement as in Figure 1
Figure 4 is a side view of the arrangement as in Figure 1 after it has been formed into its use position so as to form a Y or wishbone shape, being the position prior to fitting onto a nose;
Figure 5 is an end view of the arrangement as shown in Figure 4;
Figure 6 is a side view of the arrangement as shown in Figure 4;
Figure 7 is a perspective view of the assembly as shown in Figure 4;
Figure 8a to 8g illustrates a typical sequence of fitting instructions with steps illustrating how the nasal splint and applicator assembly of this embodiment is to be used.

### DESCRIPTION OF PREFERRED EMBODIMENTS

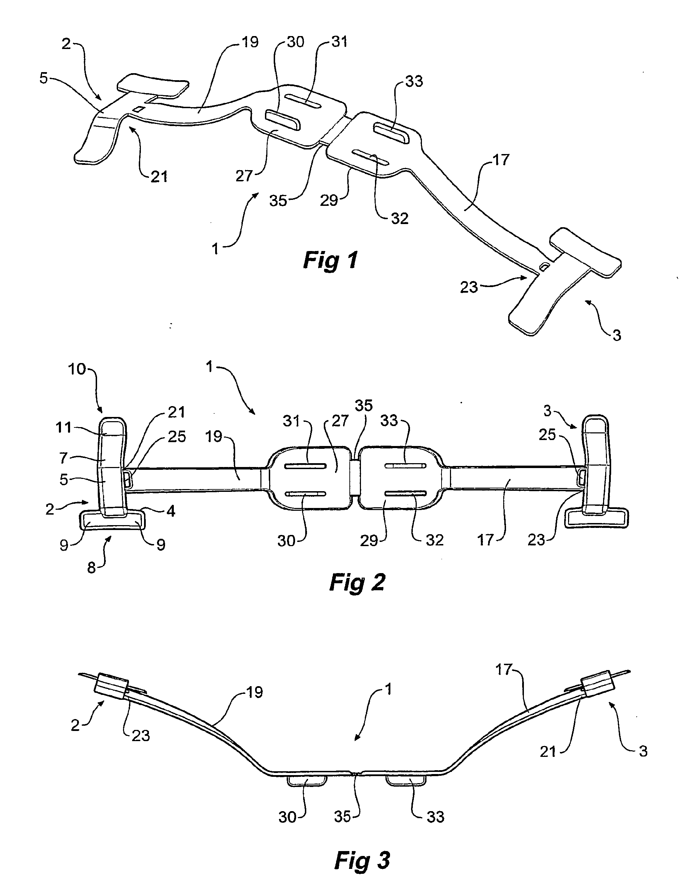

Referring in details to the drawings and in particular to Figures 1 to 7 a first embodiment of nasal splint and applicator assembly is shown.

The nasal splint and applicator assembly 1 is formed from plastics material so as to have two nasal splints 2 and 3 which are shaped so as to be attached by adhesive to one side only of a nose each and with each splint attached at one end over underlying cartilage of a nose at or toward an upper part of the nose and a further part of the splint is attached to a lower or soft tissue portion on the outside of a nasal passage so that it can be kept outwardly extended thereby to achieve nasal dilation.

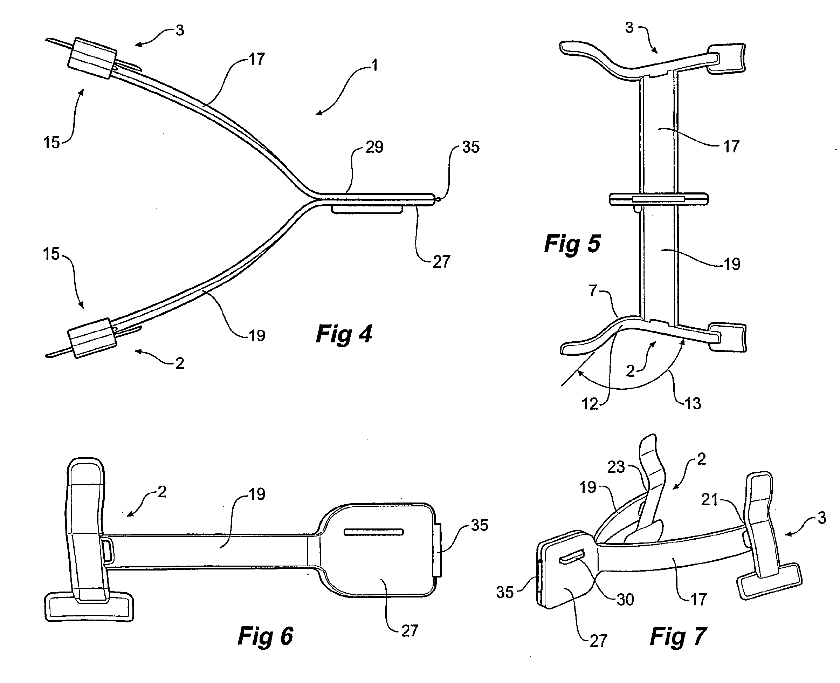

Each nasal splint has an elongate strut 5 with a bend 7 intermediate the ends. The bend has an included angle 13 (see Figure 5) on the outer side 12 of the strut of from 120° to 150°. One end 8 of the strut 5 has wings 9 to provide a greater surface of adhesive to join to the fleshy part of the nose. The other end 10 of the strut 5 has a concave bend 11 on the inside surface 15 of the strut to enhance the lever action when the strut is mounted onto the nose. Adhesive is placed on the inside surface of the strut 5 as indicated by the arrow 15 in Figure 4.

Each nasal splint has an elongate resiliently deformable strut having inner and outer sides; a bend in the strut intermediate the ends to form an included angle in the strut on the outer side and first and second portions extending from the bend; and an adhesive material on the inner side of the strut,

The aim is to get the splints into an accurate position on the nose and this is assisted by having an integrally moulded applicator assembly structure where there are two arms 17 and 19 which join the respective splints 2 and 3 at a line of weakness shown at 21 and 23 respectively. The lines of weakness 21 and 23 are further weakened by having an aperture 25 in the join and a slight narrowing of the thickness of the integral join between the handle and strut. As can be seen particularly in Figures 3 and 4 there is a twist in each of the arms 17 and 19 so that the nasal splints 2 and 3 are angled to each other when the assembly is placed into the use position so that that the struts substantially match the angles of the sides of the nose.

At a centre of the arms there is a handle portion which is formed from two grip portions 27 and 29 which have mutually corresponding projections and apertures 30 and 31 on the grip portion 27 and mutually corresponding projections and apertures 33 and 32 on the grip 29. Between the grip portions 27 and 29 is an integral hinge 35. When the handle is hinged on the integral hinge 35 so that the grip portions 27 and 29 come together the projection 30 engages into the aperture 32 and the projection 33 engages into the aperture 31 which assists in holding the grips together and in correct alignment.

Hence the assembly when moulded is in the substantially elongate shape as shown in Figures 1 to 4. For use the generally linear elongate shape of the structure as shown in Figure 1 can be folded on the integral hinge 35 so as to take up a Y or wishbone shape as is generally shown in the Figures 4 through 7.

This Y or wishbone shape is appropriate so that the arms 17 and 19 can straddle the nose with the splints 2 and 3 being then generally located on the sides of the nose in an accurate relative position, one with respect to the other.

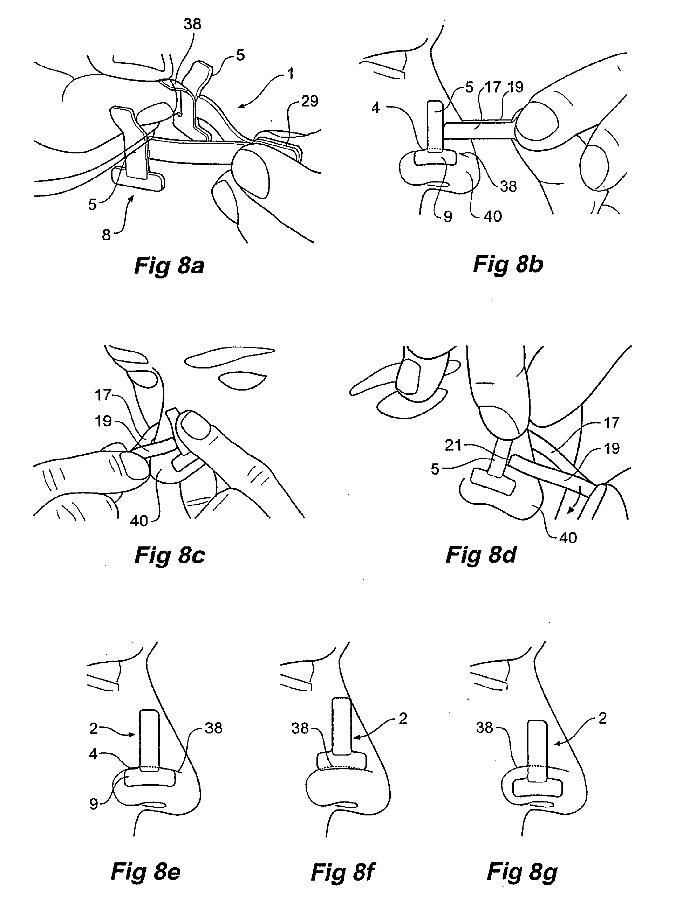

The fitting of the nasal splints is achieved in the series of steps as is shown in Figure 8a to 8g.

In Figure 8a the applicator 1 in the Y or wishbone shape is held in one hand with the fingers gripping the grips 27 and 29 and the winged ends 8 of the struts 5 directed downwardly. The covering paper 38 for the adhesive on each respective splint 7 can then be removed and this makes the assembly ready to be applied to the nose.

In Figure 8b the applicator assembly has been presented to the nose 40 with the nasal splints on either side of the nose. Each splint is aligned with the nose so that the upper edge 4 of the wings 9 aligns with the crease 38 on the upper edge of the fleshy portion of the nose as is shown in Figure 8e. The arms 17 and 19 of the applicator are then moved together with finger pressure to engage the adhesive of the splints with the sides of the nose.

As shown in Figure 8c finger and thumb pressure is then applied to the splints to firmly engage the splints with the nose 40.

While still maintaining finger and thumb pressure on the splints with the fingers a downward pull is applied to the handle and arms 17 and 19 as shown in Figure 8d so that the handle and arms are separated from the splints along the lines of weakness 21 and 23. This is relatively readily achieved by reason of the narrowing of the plastics material at this point.

Figures 8e to 8f show correct and incorrect placement of the nasal splint.

Figure 8e illustrates how the splint 2 is correctly located so that the wing portion 9 has an upper edge 4 which coincides with a crease 38 of the nose which is between the upper edge of the fleshy portion of the nose and the more rigid part of the nose.

Figures 8f and 8g show illustrations of too high and too low placement respectively. Slight changes in this relative location are acceptable.

## Claims

1. A nasal splint applicator (1) including: a handle (27,29); **characterised in that** the applicator further includes
a pair of arms (17,19) extending from the handle (27,29); and
a nasal splint (2,3) removably attached to each of the arms (17,19);
wherein the handle (27,29) and arms (17,19) form a substantially Y shape, the handle (27,29) being the base of the Y shape and the arms (17,19) forming the wings of the Y shape and each nasal splint (2,3) being adapted to be removably fastened to a respective side on the outside of the nose (40).

2. A nasal splint applicator (1) as claimed in Claim 1 **characterised in that** the nasal splints (2,3) are integrally moulded with its respective arm (17,19) with a line of weakness (21,23) between them so that the nasal splint (2,3) can be removed from the arm (17,19) by breaking at the line of weakness (21,23).

3. A nasal splint applicator (1) as claimed in Claim 1 **characterised in that** the handle (27,29) includes first and second grip portions (27,29) joined by an integral hinge (35) with respective arms (17,19) extending from respective grip portions, whereby the handle (27,29) can be moulded in a substantially elongate configuration and bent at the integral hinge (35) to form a use position of the handle (27,29).

4. A nasal splint applicator (1) as claimed in Claim 3 **characterised by** having inter-engagable portions (30,32; 31,33) on each of the first and second grip portions (27,29) which are adapted to engage when the grips (30,32; 31,33) are brought together into the handle (27,29) use position.

5. A nasal splint applicator (1) as claimed in Claim 1 **characterised in that** each nasal splint (2,3) comprises an elongate resiliently deformable strut (5) having inner and outer sides (12); a bend (7) in the strut (5) intermediate the ends to form an included angle in the strut (5) on the outer side (12) and first and second portions extending from the bend; and an adhesive material on the inner side of the strut (5), the adhesive material being adapted to be removably secured to the external skin of the nose; the splint being adapted for affixing longitudinally to only one side of the nose (40) with the first portion removably secured to a rigid fleshy portion and the second portion removably secured to a soft fleshy portion and when so affixed causes a dilation of the nasal passage as a result of a lever effect exerted on the soft fleshy portion.

6. A nasal splint applicator (1) as claimed in Claim 5 **characterised in that** the included angle on the outer side is in the range of from approximately 120 degrees to approximately 150 degrees.

7. A nasal splint applicator (1) as claimed in Claim 5 **characterised in that** the second portion of the strut (5) of the nasal splint includes side wings (9) and the adhesive material is also provided on the side wings (9).

8. A nasal splint applicator (1) as claimed in Claim 5 **characterised in that** the first portion of the strut (5) of the nasal splint includes a further bend (12) on the inner side of the strut (5).

9. A nasal splint applicator (1) as claimed in Claim 5 **characterised in that** the further bend (12) is a transverse bend in the strut (5).

10. A nasal splint applicator (1) as claimed in Claim 5 **characterised in that** the handle (27,29) is joined to the nasal splint at a position on a side of the strut (5) along the second portion.

11. A nasal splint applicator (1) as claimed in claim 1 **characterised in that** the splint (2,3) has on one face thereof means to adhere it to one side only of the nose (40) of a wearer and adapted by reason of the shape to have at least one end of the splint (2,3) adhering over an underlying more rigid portion of the structure of the nose (40).

12. A nasal splint applicator (1) as claimed in Claim 11 **characterised in that** the handle (27,29) is moulded integrally with the material of the splint (2,3) from plastics material.

13. A nasal splint applicator (1) as claimed in Claim 11 **characterised in that** the connection between the handle (27,29) and a splint (2,3) is defined by a reduced thickness portion.

14. A nasal splint applicator (1) as claimed in Claim 11 **characterised in that** the attachment includes a line of weakness (21,23) so that by levering the handle (27,29) with respect to the splint the handle (27,29) will break away.

15. A nasal splint applicator (1) as claimed in Claim 11 **characterised in that** there are two splints (2,3) secured by a handle (27,29) arrangement which has a middle portion and two arms (17,19), and the middle portion is moulded from plastics material and has a shape providing for an integral hinge (35) and two grips which are adapted to be brought together to thereby form said handle (27,29) and arms (17,19) which form a substantially Y shape.

16. A nasal splint applicator (1) as claimed in claim 1 **characterised in that** the nasal splint (2,3) comprises an elongate resiliently deformable strut (5) having inner and outer sides, a bend in the strut (5) intermediate the ends to form an included angle in the strut (5) on the outer side and first and second portions extending from the bend, the second portion of the strut (5) including side wings (9) and an adhesive material on the inner side of the strut (5) and side wings (9), the adhesive material being adapted to be removably secured to the external skin of the nose (40), the splint (2,3) being adapted for affixing longitudinally to only one side of the nose (40) with the first portion removably secured to a rigid fleshy portion and the second portion removably secured to a soft fleshy portion and when so affixed causes a dilation of the nasal passage as a result of a lever effect exerted on the soft fleshy portion by the second portion of the strut (5).

17. A nasal splint applicator (1) as claimed in Claim 16 **characterised in that** the first portion of the strut (5) of the nasal splint includes a further bend (11) on the inner side of the strut (5).

18. A nasal splint applicator (1) as claimed in Claim 16 further including a handle (27,29) joined to the splint by a line of weakness (21,23) such that after adherence of the splint to a side of the nose the handle (27,29) can be separated from the splint (2,3) by breaking along the line of weakness (23).

19. A nasal splint applicator (1) as claimed in Claim 18 **characterised in that** the handle (27,29) and splint (2,3) are integrally moulded.

## Patentansprüche

1. Nasensplint-Applikator (1) mit einem Griff (27,29), **dadurch gekennzeichnet, dass** der Applikator des Weiteren aufweist:
ein Paar Arme (17,19), die sich von dem Griff (27,29) erstrecken und
einen Nasensplint (2, 3), der abnehmbar an jedem der Arme (17,19) angebracht ist,
wobei der Griff (27,29) und die Arme (17,19) im Wesentlichen eine Y-Form ausbilden, der Griff (27,29) die Basis der Y-Form ist und die Arme (17,19) die Flügel der Y-Form bilden und jeder Nasensplint (2,3) eingerichtet ist, um jeweils an einer Seite auf der Außenseite der Nase (40) befestigt zu werden.

2. Nasensplint-Applikator (1) gemäß Anspruch 1, **dadurch gekennzeichnet, dass** die Nasensplints (2, 3) jeweils einstückig mit ihrem Arm (17,19) geformt sind und zwar mit einer linienförmigen Schwachstelle (21,23) zwischen ihnen, sodass der Nasensplint (2, 3) von dem Arm (17,19) durch Brechen an der linienförmigen Schwachstelle (21,23) entfernt werden kann.

3. Nasensplint-Applikator (1) gemäß Anspruch 1, **dadurch gekennzeichnet, dass** der Griff (27,29) einen ersten und einen zweitenGriffabschnitt (27,29) aufweist, die jeweils über ein Filmscharnier (35) mit den sich jeweils von den Griffabschnitten erstreckenden Armen (17,19) verbunden sind, wodurch der Griff (27, 29) in einer im Wesentlichen länglichen Anordnung geformt und bei dem Filmscharnier (35) gebogen werden kann, um eine Verwendungsposition des Griffs (27,29) zu bilden.

4. Nasensplint-Applikator (1) gemäß Anspruch 3, **dadurch gekennzeichnet, dass** erineinandergreifende Abschnitte (30,32; 31,33) sowohl auf dem ersten als auch auf dem zweiten Griffabschnitt (27, 29) aufweist, die eingerichtet sind, einzugreifen, wenn die Griffe (30,32; 31,33) zusammen in die Verwendungsposition des Griffs (27,29) gebracht werden.

5. Nasensplint-Applikator (1) gemäß Naspruch 1, **dadurch gekennzeichnet, dass** jeder Nasensplint (2, 3) eine längliche federnde verformbare Strebe (5) mit einer Innen- und einer Außenseite (12); eine Biegung (7) in der Strebe (5) zwischen den Enden, um einen eingeschlossenen Winkel in der Strebe (5) an der Außenseite (12)und einem ersten und zweiten Abschnitt, die sich von der Biegung erstrecken, auszubilden; und ein Haftmaterial auf der Innenseite der Strebe (5) aufweist, wobei das Haftmaterial eingerichtet ist, um abnehmbar an der äußeren Haut der Nase gesichert zu werden, der Splint zum längsseitigen Anbringen an nur einer Seite der Nase (40) eingerichtet ist und zwar mit dem ersten Abschnitt abnehmbar an einem rigiden fleischigen Abschnitt gesichert und dem zweiten Abschnitt abnehmbar an einem weichen fleischigen Abschnitt gesichert und so angebracht eine Dilatation des Nasengangs als Ergebnis einerauf den weichen fleischigen Abschnitt ausgeübten Hebelwirkung verursacht.

6. Nasensplint-Applikator (1) gemäß Anspruch 5, **dadurch gekennzeichnet, dass** der eingeschlossene Winkel an der Außenseite in dem Bereich von in etwa 120 Grad bis in etwa 150 Grad liegt.

7. Nasensplint-Applikator (1) gemäß Anspruch 5, **dadurch gekennzeichnet, dass** der zweite Abschnitt der Strebe (5) des Nasensplints Seitenflügel (9) aufweist und das Haftmaterial ebenfalls auf den Seitenflügeln (9) bereitgestellt ist.

8. Nasensplint-Applikator (1) gemäß Anspruch 5, **dadurch gekennzeichnet, dass** der erste Abschnitt der Strebe (5) des Nasensplints eine weitere Biegung (12) an der Innenseite der Strebe (5) aufweist.

9. Nasensplint-Applikator (1) gemäß Anspruch 5, **dadurch gekennzeichnet, dass** die weitere Biegung (12) eine Querbiegung in der Strebe (5) ist.

10. Nasensplint-Applikator (1) gemäß Anspruch 5, **dadurch gekennzeichnet, dass** der Griff (27,29) an einer Position auf einer Seite der Strebe (5) entlang des zweiten Abschnittsmit dem Nasensplint verbunden ist.

11. Nasensplint-Applikator (1) gemäß Anspruch 1, **dadurch gekennzeichnet, dass** der Splint (2, 3) auf einer seiner Seiten ein Mittel aufweist, um ihn auf nur einer Seite der Nase (40) eines Trägers anzuhaften, und aufgrund einer Form eingerichtet ist, um mindestens ein Ende des Splints (2, 3) über einem darunterliegenden rigideren Abschnitt der Struktur der Nase (40) haften zu lassen.

12. Nasensplint-Applikator (1) gemäß Anspruch 11, **dadurch gekennzeichnet, dass** der Griff (27, 29) einstückig mit dem Material des Splints (2, 3) aus einem Kunststoffmaterial geformt ist.

13. Nasensplint-Applikator (1) gemäß Anspruch 11, **dadurch gekennzeichnet, dass** die Verbindung zwischen dem Griff (27, 29) und einem Splint (2, 3) durch einen Abschnitt verminderter Dicke definiert ist.

14. Nasensplint-Applikator (1) gemäß Anspruch 11, **dadurch gekennzeichnet, dass** das Anbringen eine linienförmige Schwachstelle (21,23) mit einschließt, so dass durchAushebeln des Griffs (27,29) in Bezug zu dem Splint der Griff (27,29) abbricht.

15. Nasensplint-Applikator (1) gemäß Anspruch 11, **dadurch gekennzeichnet, dass** es zwei Splints (2, 3) gibt, die durch einen Griffaufbau (27, 29) gesichert sind, der einen mittleren Abschnitt und zwei Arme (17,19) aufweist, und der mittlere Abschnitt aus Kunststoffmaterial geformt ist und eine Form aufweist, die ein Filmscharnier (35) und zwei Griffe bereitstellt, die eingerichtet sind, zusammengebracht zu werden, um dadurch den Griff (27,29) und die Arme (17,19) zu bilden, die im Wesentlichen eine Y-Form bilden.

16. Nasensplint-Applikator (1) gemäß Anspruch 1, **dadurch gekennzeichnet, dass** der Nasensplint (2, 3) eine längliche federnde verformbare Strebe (5) mit einer Innen- und einer Außenseite, eine Biegung in der Strebe (5) zwischen den Enden, um einen eingeschlossenen Winkel in der Strebe (5) auf der Außenseite und einem ersten und einem zweiten Abschnitt, die sich von der Biegung erstrecken, auszubilden, wobei der zweite Abschnitt der Strebe (5) Seitenflügel (9) aufweist, und ein Haftmaterial auf der Innenseite der Strebe (5) und Seitenflügel (9) aufweist, wobei das Haftmaterial eingerichtet ist, um abnehmbar auf der äußeren Haut der Nase (40) gesichert zu sein, der Splint zum längsseitigen Anbringen an nur einer Seite der Nase (40) eingerichtet ist und zwar mit dem ersten Abschnitt abnehmbar an einem rigiden fleischigen Abschnitt gesichert und dem zweiten Abschnitt abnehmbar an einem weichen fleischigen Abschnitt gesichert undso angebrachteine Dilatation des Nasengangs als Ergebnis einerauf den weichen fleischigen Abschnitt durch den zweiten Abschnitt der Strebe (5) ausgeübten Hebelwirkung verursacht.

17. Nasensplint-Applikator (1) gemäß Anspruch 16, **dadurch gekennzeichnet, dass** der erste Abschnitt der Strebe (5) des Nasensplints eine weitere Biegung (11) auf der Innenseite der Strebe (5) aufweist.

18. Nasensplint-Applikator (1) gemäß Anspruch 16, weiterhin mit einem Griff (27,29), der mit dem Splint über eine linienförmige Schwachstelle (21,23) verbunden ist, so dass nach Anhaften des Splints an einer Seite der Nase der Griff (27,29) von dem Splint (2, 3) durch Brechen entlang der linienförmigen Schwachstelle (23) getrennt werden kann.

19. Nasensplint-Applikator (1) gemäß Anspruch 18, **dadurch gekennzeichnet, dass** der Griff (27,29) und Splint (2, 3) einstückig geformt sind.

## Revendications

1. Applicateur d'écarteur pour voies nasales (1) comprenant une poignée (27, 29), **caractérisé en ce que** l'applicateur comprend en outre :
une paire de bras (17, 19) s'étendant depuis la poignée (27, 29) ; et
un écarteur nasal (2, 3) fixé de manière amovible à chacun des bras (17, 19) ;
dans lequel la poignée (27, 29) et les bras (17, 19) ont une forme sensiblement en Y, la poignée (27, 29) étant la base de la forme en Y et les bras (17, 19) formant les ailes de la forme en Y et chaque écarteur nasal (2,3) étant adapté pour être fixé de manière amovible à un côté respectif à l'extérieur du nez (40).

2. Applicateur d'écarteur pour voies nasales (1) selon la revendication 1, **caractérisé en ce que** les écarteurs nasaux (2, 3) sont intégralement moulés avec leur bras respectif (17, 19) et présentent une ligne de faiblesse (21, 23) entre eux, de sorte que l'écarteur nasal (2, 3) puisse être enlevé du bras (17, 19) en le cassant au niveau de la ligne de faiblesse (21, 23).

3. Applicateur d'écarteur pour voies nasales (1) selon la revendication 1, **caractérisé en ce que** la poignée (27, 29) comprend une première et une seconde parties de saisie (27, 29) jointes par une articulation intégrale (35), avec des bras respectifs (17, 19) s'étendant depuis lesdites parties de saisie, moyennant quoi la poignée (27, 29) peut être moulée dans une configuration sensiblement allongée et pliée au niveau de l'articulation intégrale (35), afin de former une position d'utilisation de la poignée (27, 29).

4. Applicateur d'écarteur pour voies nasales (1) selon la revendication 3, **caractérisé en ce qu'**il présente des parties pouvant se mettre réciproquement en prise (30, 32 ; 31, 33) sur chacune de la première et de la seconde parties de saisie (27, 29), et qui sont adaptées pour se mettre en prise quand lesdites saisies (30, 32 ; 31, 33) sont mises ensemble dans la position d'utilisation de la poignée (27, 29).

5. Applicateur d'écarteur pour voies nasales (1) selon la revendication 1, **caractérisé en ce que** chaque écarteur nasal (2, 3) comprend un montant déformable élastiquement allongé (5) ayant des côtés intérieur et extérieur (12) ; une pliure (7) dans le montant (5) entre les extrémités, pour former un angle inclus dans le montant (5) sur le côté extérieur (12) et une première et une seconde parties s'étendant depuis la pliure ; et un matériau adhésif sur le côté intérieur du montant (5), le matériau adhésif étant adapté pour être fixé de manière amovible sur la peau extérieure du nez ; l'écarteur étant adapté pour se fixer longitudinalement seulement sur un côté du nez (40), la première partie étant fixée de manière amovible à une partie charnue rigide et la seconde partie fixée de manière amovible à une partie charnue souple et une fois fixé de cette façon, il provoque une dilatation des voies nasales du fait d'un effet de levier exercé sur la partie souple charnue.

6. Applicateur pour écarteur nasal (1) selon la revendication 5, **caractérisé en ce que** l'angle inclus sur le côté extérieur est dans la gamme d'environ 120 degrés à environ 150 degrés.

7. Applicateur pour écarteur nasal (1) selon la revendication 5, **caractérisé en ce que** la seconde partie du montant (5) de l'écarteur nasal comprend des ailettes latérales (9) et le matériau adhésif est également fourni sur les ailettes latérales (9).

8. Applicateur pour écarteur nasal (1) selon la revendication 5, **caractérisé en ce que** la première partie du montant (5) de l'écarteur nasal comprend une autre pliure (12) sur le côté interne du montant (5).

9. Applicateur d'écarteur pour voies nasales (1) selon la revendication 5, **caractérisé en ce que** la pliure ultérieure (12) est une pliure transversale dans le montant (5).

10. Applicateur d'écarteur pour voies nasales (1) selon la revendication 5, **caractérisé en ce que** la poignée (27, 29) est jointe à l'écarteur nasal dans une position sur un côté du montant (5) le long de la seconde partie.

11. Applicateur d'écarteur pour voies nasales (1) selon la revendication 1, **caractérisé en ce que** l'écarteur (2, 3) présente, sur une face, des moyens pour lui permettre d'adhérer à un côté seulement du nez (40) d'un patient et adapté, du fait de sa forme, pour avoir au moins une extrémité de l'écarteur (2, 3) qui adhère sur une partie plus rigide sous-jacente de la structure du nez (40).

12. Applicateur d'écarteur pour voies nasales (1) selon la revendication 11, **caractérisé en ce que** la poignée (27, 29) est intégralement moulée avec le matériau de l'écarteur (2, 3), à partir de matière plastique.

13. Applicateur d'écarteur pour voies nasales (1) selon la revendication 11, **caractérisé en ce que** la connexion entre la poignée (27, 29) et un écarteur (2, 3) est définie par une partie d'épaisseur réduite.

14. Applicateur d'écarteur pour voies nasales (1) selon la revendication 11, **caractérisé en ce que** la fixation comprend une ligne de faiblesse (21, 23) de sorte qu'en faisant levier sur la poignée (27, 29) relativement à l'écarteur, la poignée (27, 29) se rompe.

15. Applicateur d'écarteur pour voies nasales (1) selon la revendication 11, **caractérisé en ce qu'**il existe deux écarteurs (2, 3) fixés par un dispositif à poignée (27, 29), doté d'une partie centrale et de deux bras (17, 19) et la partie centrale est moulée à partir de matière plastique et a une forme permettant d'obtenir une articulation intégrale (35) et deux prises qui sont adaptées pour être mises ensemble, afin de former ainsi ladite poignée (27, 29) et les bras (17, 19) qui forment une forme sensiblement en Y.

16. Applicateur d'écarteur pour voies nasales (1) selon la revendication 1, **caractérisé en ce que** l'écarteur nasal (2, 3) comprend un montant déformable élastiquement allongé (5), ayant des côtés interne et externe, une pliure dans le montant (5) entre les extrémités pour former un angle inclus dans le montant (5) sur le côté extérieur et une première et seconde parties s'étendant depuis la pliure, la seconde partie du montant (5) comprenant des ailettes latérales (9) et un matériau adhésif sur le côté interne du montant (5) et des ailettes latérales (9), le matériau adhésif étant adapté pour être fixé de manière amovible à la peau extérieure du nez (40), l'écarteur (2, 3) étant adapté pour se fixer longitudinalement seulement à un côté du nez (40), la première partie étant fixée de manière amovible à une partie charnue rigide et la seconde partie étant fixée de manière amovible à une partie souple charnue et lorsqu'il est fixé ainsi, il provoque une dilatation des voies nasales du fait d'un effet de levier exercé sur la partie souple charnue par la seconde partie du montant (5).

17. Applicateur d'écarteur pour voies nasales (1) selon la revendication 16, **caractérisé en ce que** la première partie du montant (5) de l'écarteur nasal comprend une autre pliure (11) sur le côté interne du montant (5).

18. Applicateur d'écarteur pour voies nasales (1) selon la revendication 16, comprenant en outre une poignée (27, 29) jointe à l'écarteur par une ligne de faiblesse (21, 23), de sorte qu'après adhésion de l'écarteur sur un côté du nez, la poignée (27, 29) peut être séparée de l'écarteur (2, 3), en la rompant au niveau de la ligne de faiblesse (23).

19. Applicateur d'écarteur pour voies nasales (1) selon la revendication 18, **caractérisé en ce que** la poignée (27, 29) et l'écarteur (2, 3) sont intégralement moulés.
